# EUROPEAN PATENT SPECIFICATION

(11) **EP 0 655 087 B1**
(45) Date of publication and mention of the grant of the patent: **03.09.1997**
(21) Application number: 92921899.8
(22) Date of filing: 09.10.1992
(51) Int. Cl.: C12P 7/62

(54) **PROCESS FOR THE PREPARATION OF AROMA ESTERS BY AN ENZYME CATALYTIC REACTION**
VERFAHREN ZUR HERSTELLUNG VON AROMAESTERN DURCH EINE ENZYMATISCH KATALYSIERTE REAKTION
PROCEDE DE PREPARATION D'ESTERS AROMATIQUES PAR UNE REACTION CATALYSEE PAR UN ENZYME

(30) Priority: 05.08.1992 HU 9202542
(43) Date of publication of application: 31.05.1995
(73) Proprietor: NITROIL VEGYIPARI TERMELÖ-FEJLESZTÖ RT., H-8105 Varpalota (HU); MTA Müszaki Kémiai Kutato Intézet, H-8201 Veszprém (HU)
(72) Inventor: GUBICZA, László, H-8200 Veszprém (HU); SZAKACSNE SCHMIDT, Aniko, H-8200 Veszprém (HU); EISENREICH, Zoltánné, H-8200 Veszprém (HU); UJHIDY, Aurél, H-8200 Veszprém (HU); HODOSSY, Lajos, H-8200 Veszprém (HU); GEMES, István, H-8100 Várpalota (HU); DROZDA, Tamás, H-8200 Veszprém (HU)
(74) Representative: Beszédes, Stephan G., Dr.
(86) International application number: HU9200038
(87) International publication number: WO9403623

(56) References cited:
- EP-A- 0 061 023
- EP-A- 0 190 651
- Biotechnology Letters, Volume 13, No. 1, issued January 1991 (Elsevier, Amsterdam), A. MANJON et al., "Short-Chain Flavour Ester Synthesis by Immobilized Lipaso in Organic Media", see pages 339-344.

## Description

The invention relates to a process for the preparation of aroma esters (i.e. C₂₋₆ aliphatic esters of C₂₋₆ carboxylic acids) by esterifying the respective C₂₋₆ aliphatic carboxylic acids with the respective C₂₋₆ aliphatic alcohols in an organic solvent in the presence of an enzyme catalyst.

The natural or nature-like aroma esters prepared according to the invention can be utilized, for example, in food industry.

In modern food industrial techniques foods and beverages should frequently be completed with aroma substances, since aroma, flavour and odour substances originally present may damage under the conditions of foodstuff production and/or preservation, or a part of them gets lost. Although a part of the lost aroma substances can be recovered and, after appropriate enrichment steps, returned into the products, due to the relatively low efficiency of the recovering and enrichment operations it is advisable to complete them with natural or nature-like aroma components in order to supplement the losses.

Methods for preparing aroma concentrates can be classified into the following three main groups: distillation or rectification; adsorption followed by desorption; extraction. The process steps and their combinations applied to perform these methods are selected so that losses in aroma components can be suppressed to the minimum. However, even when operating with maximum care, the original combination of aroma substances can never be reproduced in the resulting concentrates; thus e.g. if a fruit juice is applied as starting substance, the ratio of the individual aroma components in the resulting concentrate will always differ in more or less extent, depending on the type and quality of the starting fresh fruit juice and on the combination of operations applied, from that observed in the fresh juice. These differences are not only due to the inevitable losses occurring in the various physical operations but undesired decomposition processes can be detected, too (Hochberg: Getränkeindustrie 9, 784 /1986/).

Aroma substances and aroma components produced by biotechnological methods from reactants of natural origin under applying naturally occurring reaction aids (such as solvents, catalysts etc.) or under ensuring that non-natural components do not appear in the product are regarded as natural substances in most of the countries. From biotechnological aspects the methods for producing such nature-like substances can be classified into the following two main groups: microbiological reactions and enzymatic reactions (Welsh et al.: Critical Rev. Biotechn. 9, 105 /1989/).

The main disadvantage of microbiological methods is that organoleptic substances, flavour and aroma substances appear in extremely low concentrations in the fermentation broth, thus their enrichment and isolation in appropriately pure state is highly expensive. In principle, it is possible to increase the amount of certain aroma substances formed in fermentation processes by selecting appropriate microorganisms or by subjecting the strains to genetic manipulations, such solutions are, however, not yet applicable on industrial scale.

According to a method developed by Novo Laboratories natural aroma and flavour substances are prepared by reacting the respective alcohols and carboxylic acids in a non-aqueous medium in the presence of lipase of Rhizopus arrhizus origin. The esters are obtained with a yield of 30-80 %. When applying a mixture of tert.butyl methyl ether and acetone as solvent only primary alcohols and fatty acids above 10 carbon atoms could be esterified with appropriate yields (Gancet et al.: Rev. Fr. Corps. GRAS 33, 423 /1986/).

The above method is applicable primarily for the preparation of higher alkyl higher carboxylates, and appropriate results can be expected with normal (straight-chained) alcohols. Fruit aroma substances contain, however, numerous esters formed from lower alcohols, both straight-chained and branched ones, and lower carboxylic acids (such as ethyl butyrate, isoamyl butyrate, isobutyl butyrate, etc.). The preparation of such esters in non-aqueous media via enzyme catalysis is described by Gatfield (Ann. Y. N. Acad. Sci. 436, 569 /1984/; Lebensm. Wiss. u. Technol. 19, 87 /1986/). The author has examined the esterification of various acids (such as propionic, valeric, butyric and caprylic acids) with C₁₋₁₀ alcohols under lipase catalysis, applying lipase of Mucor miehei origin, and has found that under identical reaction conditions C₁₋₄ alkyl propionates and butyrates can be prepared only with a yield below 30 %. Other authors have also reported on the preparation of natural aroma esters, emphasizing that the use of an immobilized lipase preparation of Candida cylindracea origin enables one to produce the required products with higher reaction rates and in better yields than those attainable with non-immobilized enzyme, and immobilization renders the enzyme usable in several cycles. Upon examining the effects of substrate concentration the authors have found that, due to the appearance of substrate inhibition, it is not advisable to raise the concentration of ethanol above 1.2 moles/litre and that of butyric acid above 0.5 mole/litre (Gilles et al.: Biocatalysis in Organic Media p. 227 /Elsevier, Amsterdam, 1987/; Biotechnol. Lett. 9, 709 /1987/).

Murray et al. (Proceedings of the 5th Intern. Flavor Conf. Porto Karras Chalkidiki, Greece, 1-3 July 1987) investigated the formation of esters of low molecular weight by reacting the respective natural carboxylic acids and alcohols, prepared by fermentation, in hexane as solvent. Of the lipases utilized as catalysts porcine pancreatic lipase and yeast (Candida cylindracea) lipase proved to be the most appropriate in the preparation of butyl butyrate; approximately full conversion could be attained in a 24 hours' reaction. Porcine pancreatic lipase proved to be the most appropriate catalyst in the preparation of ethyl butyrate; a conversion of 90 % could be attained in a 48 hours' reaction.

Fusel oils contain a considerable amount of C₂₋₅ alcohols. Welsh et al. (J. Food Sci. 54, 1564 /1989/) reported on the esterification of a fusel oil comprising as major components propanol (2.3 g/100 g of fusel oil), isobutanol (17.8 g/100 g) and isopentanol (79.7 g/100 g) with acetic acid or butyric acid, respectively. When performing the reaction at 30°C in hexane as solvent in the presence of immobilized yeast (Candida cylindracea) lipase butyrates were obtained with a yield of 65.8 %, whereas the yield of the respective acetates was only 46.4 %. The immobilized enzyme could be applied in three cycles for the esterification of acetic acid without loss of activity, whereas when esterifying butyric acid the enzyme activity decreased considerably even after the first cycle. The authors presented as a particularly remarkable fact that when acetic acid is esterified the acid concentration causing substrate inhibition is 1/7 of that observed with butyric acid, furthermore when butyric acid is esterified the ester yield decreases considerably if acetic acid is also present even in low amounts.

In a subsequent publication (J. Food Sci. 55, 1679 /1990/) it is reported that esters of low molecular weight can be prepared with particularly good conversion when a lipase of Candida cylindracea, Pseudomonas fluorescens or Mucor miehei origin is applied as catalyst. Of the solvents examined in a reaction performed at 30°C methylene chloride proved to be practically inapplicable. Hexane, octane and decane proved to be equally well applicable in the preparation of isopentyl butyrate and butyl butyrate, whereas the applicability of these solvents decreased in the given order in the preparation of isopentyl acetate and ethyl butyrate. With lipase of Pseudomonas fluorescens origin substrate inhibition appeared at acid concentrations above 0.5 mole/litre, whereas with lipases of Candida cylindracea and Mucor miehei origin substrate inhibition appeared only at acid concentrations exceeding 1 mole/litre.

A further publication of the same authors (Enzyme Microb. Techn. 2, 743 /1990/) deals with the use of lipases of porcine pancreas, Candida cylindracea and Aspergillus niger origin in the synthesis of ethyl and butyl butyrate performed in hexane at 30°C or 50°C, respectively. The authors have found that the degree of substrate inhibition and the limiting concentration vary with the enzyme utilized (the limiting concentration varies between 0.6 and 1.0 mole/l), and are also dependent on the ester obtained. The effect of temperature change was different in tendency with the two esters examined. The authors have found that the reaction rate decreases upon dehydrating the substrates with a molecular sieve 3 Å in pore diameter. Depending on the enzyme utilized, the esters have been obtained with a maximum yield of 67 to 83 % within a 24 hours' reaction.

Manjón et al. (Biotechnol. Lett. 13, 339 /1991/) have examined the preparation of lower aroma esters utilizing Mucor miehei lipase as catalyst. The authors have found that the conversion is highly dependent on the nature of the solvent applied. The most favourable results were obtained with solvents with log P values (the logarithm of the distribution coefficient of the solvent in a n-octane/water biphasic system) of 2 to 4.5; solvents of this type are n-hexane, n-octane and i-octane. The optimum temperature of the reaction was found to be 30-50°C. Upon examining the role of water the authors have found that the water content of the reaction mixture should be below 1 %. Conversions of 97 %, 98 % and 96 % could be attained for ethyl propionate, ethyl butyrate and ethyl hexanoate, respectively, but, due to substrate inhibition, the starting concentration of the acid always had to be kept below 0,1 mole/litre.

All of the above papers reported on the appearance of substrate inhibition with each of the enzymes examined. Substrate inhibition was caused primarily by the acid, and its extent was reported to be particularly high with acetic acid, one of the most important reactant in the preparation of fruit aroma esters. Although the extent of substrate inhibition was found to decrease with the increase of the number of carbon atoms in the acid, high conversions could be attained only with a starting acid concentration of 0,1-0,5 mole/litre, thus rather dilute mixtures had to be manipulated in the processing of the reaction mixture. The reactions reported in the references cited above were performed on a small scale, utilizing generally 10-50 ml of solvent, 0,025-0,1 mole of acid, a generally twofold excess of alcohol and 1-5 % by weight, calculated for the weight of the reaction mixture, of enzyme. The reaction time required to attain a conversion of 60-97 % was particularly high (22-48 hours).

Only a minor attention has been paid to water which forms as by-product in esterification. It is well known that in esterifications high yields can be expected generally when water, formed in the reaction as by-product, is removed. A minimum amount of water should, however, be present by all means in the reaction mixture, since otherwise the hydrolases are unable to function in organic solvents. Therefore it is not suitable to add a molecular sieve to the reaction mixture, since it absorbs not only the water formed in the reaction but also deprives the enzyme from the minimum amount of water indispensable for enzyme functioning.

The invention aims at the elaboration of a new method which enables one to prepare aroma esters on industrial scale within a shorter reaction time and in much higher concentrations than before.

It has been found that these requirements can be met completely when the starting acid content of the reaction mixture is adjusted to the highest possible value which does not cause substrate inhibition, and the acid and alcohol consumed in the reaction are supplemented continuously at the rate of their consumption. In this way a high ester concentration can be attained in the reaction mixture. Unlike acids, esters do not inhibit enzyme functions (i.e. they do not exert product inhibition), thus their concentration in the reaction mixture may be the multiple of that corresponding to the low starting acid concentration to be adjusted in order to avoid substrate inhibition. By supplementing continuously the alcohol at the rate of its consumption not only the required reactant is provided for but the optimum acid:alcohol ratio can also be maintained throughout the whone reaction. Upon these measures the initial reaction rate increases, and, since the substrate concentration and the ratio of the reactants are constant, a steady reaction rate can be maintained. A favourable consequence of this steady reaction rate is that the rate of water formation is steady, too. This enables one to remove the water formed in the reaction continuously, at the rate of its formation, e.g. in such a way that the reaction mixture is passed through a solid or liquid desiccant at a rate enabling the removal of water formed as by-product only, but leaving in the mixture the amount of water indispensable for enzyme functioning. Thus, according to the method of the invention, optimum parameters can be maintained throughout the whole reaction. This results in a considerable increase in reaction rate and in a considerable decrease in reaction time required to attain a pre-determined conversion.

The water content of the enzyme, that of the solvent and that of the substrates make up the starting water content of the reaction mixture. The water content should be adjusted so that optimum conditions for enzyme functions are provided for. As it has been mentioned above, the enzyme is unable to function in a completely anhydrous mixture, whereas if the water content is too high, the thermodynamic equilibrium of the reaction is shifted to hydrolysis. Therefore the starting water content of the reaction mixture should be adjusted to 0.01-2.0 % by weight, preferably to 0.05-1.0 % by weight, and should be maintained within these limits throughout the whole reaction.

Based on the above, the invention relates to a process for the preparation of aroma esters by esterifying a C₂₋₆ aliphatic carboxylic acid or a mixture of such acids with a C₂₋₆ aliphatic alcohol or with a mixture of such alcohols in an organic solvent at a temperature of 5-70°C in the presence of a hydrolase enzyme which is stable in the solvent applied, under maintaining the acid : alcohol molar ratio between 1:0.5 and 1:10. According to the invention the initial acid content of the reaction mixture is adjusted to 75-99 % of the acid concentration which causes substrate inhibition, the acid and the alcohol consumed in the reaction are supplemented continuously, at a rate of their consumption, and the water content of the reaction mixture is maintained at 0.01-2.0 % by weight throughout the whole reaction either by passing the reaction mixture continuously and at a controlled rate through a solid or liquid desiccant, or by subjecting a portion of the reaction mixture to azeotropic distillation to remove water and supplementing the solvent removed.

According to a preferred method of the invention the reaction mixture is continuously passed through a desiccant, preferably through a column filled with 4A zeolite, for the continuous removal of water formed as by-product in the reaction. Flow rate should be adjusted to a value which enables the removal of the water formed as by-product only, leaving thereby the starting water content of the mixture, adjusted to the optimum, within the optimum range. The appropriate flow rate can be determined easily on the basis of routine preliminary experiments.

As mentioned above, the substrates of esterification, i.e. the acid component and the alcohol component, may be a single acid or a mixture of acids and a single alcohol or a mixture of alcohols. Straight-chained and branched acids and alcohols can equally be applied. The alcohol may be either a primary or a secondary one. The acid : alcohol molar ratio may be varied within a wide range, however, in order to ensure an appropriate reaction rate, it is not advisable to decrease the acid : alcohol molar ratio below 1:0.5. In some instances the alcohol reactant may also serve as solvent for the reaction. The preferred acid : alcohol molar ratio is a value between 1:1 and 1:6.

All of the hydrolases capable of catalyzing the esterification of C₂₋₆ carboxylic acids with C₂₋₆ alcohols can be applied as enzyme catalysts in the reaction. Lipases (E.C. 3.1.1.3.), such as lipase of Mucor miehei, Candida cylindracea, Rhizopus arrhizus, Aspergillus niger, Pseudomonas fluorescens and porcine pancreas origin, and esterases (E.C. 3.1.1.1.), such as an esterase extracted from porcine liver, can be utilized to advantage. The enzymes can be applied either as such or in immobilized forms.

As reaction medium it is preferred to apply a hydrophobic organic solvent which does not deprive the enzyme from the indispensible amount of water, and in which the starting C₂₋₆ carboxylic acids and C₂₋₆ alcohols are appropriately soluble. Mixtures of such solvents can be utilized as well. Of the appropriate solvents e.g. C₅₋₁₀ paraffinic hydrocarbons (such as hexane, octane, decane), esters of the starting acid (such as ethyl acetate), methyl cyclohexane and methyl ethyl ketone are to be mentioned. It should also be taken into account as an important feature that the enzyme to be applied should be stable in the selected solvent or solvent mixture.

The enzymes exert their activity in such solvents at 5-70°C. Upon considering both the requirement for high reaction rate and the possible deactivation of the enzyme at higher temperatures it is preferred to adjust the reaction temperature to 20-55°C.

Enzyme-catalyzed esterification can be performed both in a continuous and in a semi-continuous way. According to a preferred method one proceeds as follows:

The acid content of the starting reaction mixture is adjusted to the highest permissible value which does not cause substrate inhibition. The reactants and the solvent are dehydrated, if necessary, or the optimum water content is adjusted by adding water to the mixture. The required amount of enzyme is added to the mixture under continuous stirring, and the reaction mixture is maintained at the prescribed temperature. Together with the start of the reaction the introduction of alcohol and acid is also started in order to supplement the reactants consumed. The alcohol and the acid can be introduced either separately or as an equimolar mixture of the two reactants, provided that they are appropriately miscible with one another. The rate of introduction is adjusted so that only the consumed amounts of alcohol and acid are supplemented, and substrate inhibition due to an over-addition of the acid should by all means be avoided. The progress of the reaction can be monitored by any appropriate analytical method, such as by gas chromatography. After the start of the reaction it is preferred to start immediately the continuous removal of water, formed as by-product, from the mixture. For this purpose any method of dehydration can be applied which does not interfere with the reaction and does not introduce any contamination into the reaction misture. According to a preferred method water formed as by-product in the reaction is absorbed on 3A or 4A zeolite. The reaction mixture is passed through a column filled with zeolite at a rate which enables the absorption of just the amount of water formed as by-product. Exhausted zeolite can be regenerated by heat treatment at a high temperature. When the addition of acid and alcohol is stopped, it is preferred to allow the mixture to react further in order to convert the majority of alcohol and acid still present into the required ester. In this way the losses can be decreased and the processing of the reaction mixture can be simplified.

The reaction mixture can be processed by any method suitable to separate the ester(s) formed from the solvent and the residual alcohol and acid. When the mixture is processed by distillation, azeotropic mixtures are frequently obtained. An azeotropic mixture comprising the solvent and the reactants (or the ester product) can be introduced, however, into a subsequent operation as starting substance, after readjusting the amounts of the individual components to the appropriate values.

The invention is elucidated in detail by the aid of the following non-limiting Examples. In the Examples solvents and reactants of reduced water content were applied. As enzyme either Lipozyme IM 60 (a lipase of Mucor miehei origin, produced by Novo Industri A/S) or yeast (Candida cylindracea) lipase produced by Sigma Co. was applied.

### Example 1

24.0 g (0.4 mole) of acetic acid, 105.8 g (1.2 moles) of isoamyl alcohol and 1800 ml of n-hexane are introduced into a flask of 5 litres capacity, equipped with a stirrer and with a reflux condenser. The water content of the reaction mixture is adjusted to 0.4 % by weight by introducing 5.0 g of distilled water, and the reaction mixture is heated to 40°C under stirring. Thereafter 60 g of Lipozyme IM 60 are added to the mixture, whereupon esterification sets in. Simultaneously the reaction mixture is started to circulate by a peristaltic pump through a column of 2 litres capacity, filled with 4A zeolite. The rate of circulation is adjusted so that the water content of the reaction mixture always remains 0.3-0.5 % by weight. Together with the introduction of the enzyme the continuous introduction of acetic acid and isoamyl alcohol is started in order to supplement the reactants consumed. The reactants are introduced as separate streams by metering pumps. 216.2 g (3.6 moles) of acetic acid and 317.3 g (3.6 moles) of isoamyl alcohol are introduced within a total reaction time of 24 hours. After 24 hours of reaction the addition of the reactants is stopped, and the mixture is reacted for further 2 hours. 481.7 g of isoamyl acetate are obtained; this corresponds to a yield of 92.5 % related to the total amount of acetic acid introduced.

### Example 2

One proceeds as described in Example 1 with the difference that 88.9 g (1.2 moles) of isobutanol are applied instead of the isoamyl alcohol, and further 266.8 g (3.6 moles) of isobutanol are introduced into the reaction mixture within the 24 hours' reaction time. 423.7 g (91.2 %) of isobutyl acetate are obtained.

### Example 3

One proceeds as described in Example 1 with the difference that 55.3 g (1.2 moles) of ethanol are applied instead of the isoamyl alcohol, and further 216.2 g (3.6 moles) of acetic acid and 165.8 g (3.6 moles) of ethanol are added to the reaction mixture within a 40 hours' reaction time. The mixture is reacted further for 5 hours. 304.8 g (86.5 %) of ethyl acetate are obtained.

### Example 4

One proceeds as described in Example 1 with the difference that ethyl acetate is applied as solvent instead of n-hexane. At the end of the reaction 462.4 g (88.8 %) of isoamyl acetate are obtained.

### Example 5

One proceeds as described in Example 1 with the difference that 35.3 g (0.4 mole) of isoamyl alcohol are added to the starting reaction mixture. 145.8 g (28.0 %) of isoamyl acetate are obtained.

### Example 6

One proceeds as described in Example 1 with the difference that 211.6 g (2.4 moles) of isoamyl alcohol are added to the starting reaction mixture. 330.2 g (63.4 %) of isoamyl acetate are obtained.

### Example 7

One proceeds as described in Example 1 with the difference that 48.0 g (0.8 mole) of acetic acid are added to the starting reaction mixture, and no further reactants are introduced into the mixture. After 24 hours of reaction 11.8 g (11.3 %) of isoamyl acetate are obtained.

### Example 8

One proceeds as described in Example 1 with the difference that 66.7 g (0.9 mole) of propionic acid and 317.3 g (3.6 moles) of isoamyl alcohol are added to the starting reaction mixture, and 1800 ml of methyl cyclohexane are applied as solvent. 40 g of enzyme are introduced, and further 266.7 g (3.6 moles) of propionic acid and 317.3 g (3.6 moles) of isoamyl alcohol are introduced continuously into the reaction mixture within 16 hours. The mixture is reacted further for 3 hours. 631.5 g (97.3 %) of isoamyl propionate are obtained.

### Example 9

One proceeds as described in Example 1 with the difference that 79.3 g (0.9 mole) of butyric acid and 216.4 g (3.6 moles) of n-propanol are added to the starting reaction mixture, and 1800 ml of methyl cyclohexane are applied as solvent. 40 g of enzyme are introduced, and further 317.2 g (3.6 moles) of butyric acid and 216.4 g (3.6 moles) of n-propanol are added continuously to the reaction mixture within 16 hours. The mixture is reacted further for 3 hours. 546.6 g (93.3 %) of n-propyl butyrate are obtained.

### Example 10

One proceeds as described in Example 1 with the difference that 79.3 g (0.9 mole) of butyric acid and 367.8 g (3.6 moles) of hexanol are added to the starting reaction mixture, and 1800 ml of n-octane are applied as solvent. 40 g of enzyme are introduced, and further 317.2 g (3.6 moles) of butyric acid and 367.8 g (3.6 moles) of hexanol are added continuously to the reaction mixture within 16 hours. The mixture is reacted further for 3 hours. 670.5 g (86.5 %) of hexyl butyrate are obtained.

### Example 11

One proceeds as described in Example 1 with the difference that 104.5 g (0.9 mole) of hexanecarboxylic acid and 317.3 g (3.6 moles) of isoamyl alcohol are added to the starting reaction mixture and 1800 ml of methyl cyclohexane are applied as solvent. 40 g of enzyme are introduced, and further 418.0 g (3.6 moles) of hexanecarboxylic acid and 317.3 g (3.6 moles) of isoamyl alcohol are added continuously to the reaction mixture within 16 hours. The mixture is reacted further for 3 hours. 564.2 g (67.3 %) of isoamyl hexanoate are obtained.

### Example 12

One proceeds as described in Example 1 with the difference that 66.7 g (0.9 mole) of propionic acid, 266.8 g (3.6 moles) of 2-butanol and 40 g of yeast (Candida cylindracea) lipase are added to the starting reaction mixture. Further 207.4 g (2.8 moles) of propionic acid and 207.5 g (2.8 moles) of sec.butanol are added to the reaction mixture continuously within 16 hours. The mixture is reacted further for 3 hours. 422.4 g (87.7 %) of see.butyl propionate are obtained.

### Example 13

The enzyme used in Example 1 is removed by filtration and reused in further cycles under identical conditions. In the second cycle 476.0 g (91.4 %), in the third cycle 459.0 g (88.2 %), whereas in the fourth cycle 418.1 g (80.3 %) of isoamyl acetate are obtained.

### Example 14

One proceeds as described in Example 1 with the difference that the reaction temperature is adjusted to 50°C. 486.4 g (93.4 %) of isoamyl acetate are obtained. The enzyme is removed by filtration and reused in further cycles under identical conditions. In the second cycle 457.7 g (87.9 %) and in the third cycle 422.3 g (81.1 %) of isoamyl acetate are obtained.

### Example 15

One proceeds as described in Example 1 with the difference that 98.7 g of fusel oil, comprising 70.5 g (0.8 mole) of isoamyl alcohol, 22.2 g (0.3 mole) of isobutanol and 6.0 g (0.1 mole) of n-propanol, are added to the starting reaction mixture instead of the isoamyl alcohol. Further 296.1 g of fusel oil are introduced into the reaction mixture during the 24 hours' reaction. The resulting product comprises 299.4 g of isoamyl acetate, 104.5 g of isobutyl acetate and 30.6 g of propyl acetate; the yield of the esters is 87.5 % related to the total amount of acetic acid introduced.

### Example 16

One proceeds as described in Example 1 with the difference that 79.3 g (0.9 mole) of butyric acid and 266.8 g (3.6 moles) of isobutanol are added to the starting reaction mixture, and 1800 ml of methyl ethyl ketone are applied as solvent. 40 g of yeast (Candida cylindracea) lipase are added to the mixture, and further 317.2 g (3.6 moles) of butyric acid and 266.8 g (3.6 moles) of isobutanol are introduced into the mixture during the 24 hours' reaction time. The mixture is reacted further for 5 hours. 551.0 g (84.9 %) of isobutyl butyrate are bbtained.

### Example 17

One proceeds as described in Example 1 with the difference that 10.2 g of water are added to the starting reaction mixture to adjust its water content to 0.8 % by weight, and the water content of the mixture is maintained at this value throughout the reaction. 398.4 g (76.5 %) of isoamyl acetate are obtained.

### Example 18

One proceeds as described in Example 1 with the difference that no water is added to the starting reaction mixture, and no further reactants are introduced. After a reaction time of 24 hours 11.5 g (22.0 %) of isoamyl acetate are obtained.

### Example 19

One proceeds as described in Example 1 with the difference that 2.4 g of water are added to the starting reaction mixture to adjust its water content to 0.2 % by weight, and the water content of the mixture is maintained at this value throughout the reaction. 424.9 g (81.6 %) of isoamyl acetate are obtained.

### Example 20

One proceeds as described in Example 1 with the difference that 15.3 g of water are added to the starting reaction mixture to adjust its water content to 1.2 % by weight, and the water content of the mixture is maintained at this value throughout the reaction. 352.6 g (66.7 %) of isoamyl acetate are obtained.

## Claims

1. A process for the preparation of aroma esters by esterifying a C₂₋₆ aliphatic carboxylic acid or a mixture of such acids with a C₂₋₆ aliphatic alcohol or with a mixture of such alcohols in an organic solvent at a temperature of 5-70°C in the presence of a hydrolase enzyme which is stable in the solvent applied, under maintaining the acid : alcohol molar ratio between 1:0.5 and 1:10, characterized in that the initial acid content of the reaction mixture is adjusted to 75-99% of the acid concentration which causes substrate inhibition, the acid and the alcohol consumed in the reaction are supplemented continuously, at a rate of their consumption, and the water content of the reaction mixture is maintained at 0.01-2.0 % by weight throughout the whole reaction either by passing the reaction mixture continuously and at a controlled rate through a solid or liquid desiccant, or by subjecting a portion of the reaction mixture to azeotropic distillation to remove water and supplementing the solvent removed.

2. A process as claimed in claim 1, characterized in that the water content of the reaction mixture is maintained at 0.05-1.0 % by weight.

## Patentansprüche

1. Verfahren zur Herstellung von Aromaestern durch Esterifizierung einer C₂₋₆ aliphatischen Carbonsäure oder eines Gemisches von solchen Säuren mit einem C₂₋₆ aliphatischen Alkohol oder einem Gemisch von solchen Alkoholen in einem organischem Lösungsmittel bei einer Temperatur von 5-70 °C, in Gegenwart eines Hydrolase-Enzyms, welches in dem verwendeten Lösungsmittel stabil ist, unter Aufrechterhaltung des molaren Verhältnisses der Säure und des Alkohols zwischen 1:0,5 und 1:10, dadurch **gekennzeichnet**, dass der anfängliche Säuregehalt des Reaktiongemisches auf 75-99% der Säurekonzentration, die Substratinhibierung hervorruft, eingestellt wird, und die in der Reaktion verbrauchte Säure und Alkohol im Verhältnis ihres Verbrauches kontinuierlich ergänzt werden, und der wassergehalt des Reaktionsgemisches während der ganzen Reaktion bei einem Wert von 0,01-2,0 Gewicht% gehalten wird entweder durch kontinuierliche Durchleitung des Reaktionsgemisches bei kontrollierter Geschwindigkeit durch einen festen oder flüssigen Dessikator oder durch Unterwerfen eines Teiles des Reaktionsgemisches einer azeotropen Destillation zwecks Entfernung des Wassers und Ergänzung des entfernten Lösungsmittels.

2. Verfahren nach Anspruch 1, dadurch gekennzeichnet, dass der Wassergehalt des Reaktionsgemisches bei 0,05-1,0 Gewichts% gehalten wird.

## Revendications

1. Procédé pour la préparation d'esters aromatiques par l'estérification d'un acide carboxylique aliphatique en C₂₋₆ ou d'un mélange de tels acides avec un alcool aliphatique en C₂₋₆ ou avec un mélange de tels alcools dans un solvant organique à une température de 5-70 °C en la présence d'une enzyme d'hydrolase qui est stable dans le solvant mis en oeuvre, tout en maintenant le rapport molaire acide:alcool entre 1:0,5 et 1:10, caractérisé en ce que le contenu acide initial du mélange réactionnel est ajusté à 75-99 % de la concentration acide qui provoque l'inhibition de substrat, l'acide et l'alcool consommés durant la réaction sont ajoutés continuellement au rythme de leur consommation, et la proportion de l'eau dans le mélange réactionnel est maintenue à 0,01-2,0 % en masse durant toute la réaction soit en faisant passer le mélange réactionnel à un rythme contrôlé et continuellement par un dessiccatif solide ou liquide, soit en soumettant une partie du mélange réactionnel à une distillation azéotrope afin d'en éliminer l'eau et d'y ajouter le solvant éliminé.

2. Procédé selon la revendication 1, caractérisé en ce que la proportion de l'eau dans le mélange réactionnel est maintenue à 0,05-1,0 % en masse.
